(19) Europäisches Patentamt
European Patent Office
Office européen des brevets

(11) Publication number: **0 477 454 A1**

# (12) EUROPEAN PATENT APPLICATION

(21) Application number: **90402695.2**

(22) Date of filing: **28.09.90**

(51) Int. Cl.$^5$: **C07H 19/10**, C07H 19/20, A61K 31/70

(43) Date of publication of application:
**01.04.92 Bulletin 92/14**

(84) Designated Contracting States:
**FR**

(71) Applicant: **MERRELL DOW PHARMACEUTICALS INC.**
**2110 East Galbraith Road**
**Cincinnati Ohio 45215-6300(US)**

(72) Inventor: **Halazy, Serge**
**2 Rue Hans Haug, Wolfisheim**
**F-67200 Strasbourg(FR)**
Inventor: **Casara, Patrick**
**12 Rue de la Scierie**
**F-67117 Ittenheim(FR)**
Inventor: **Neises, Bernhard**
**Flösserweg 5**
**W-7600 Offenburg-Griesheim(DE)**
Inventor: **Jund, Karin**
**7 Boulevard Clémenceau**
**F-67000 Strasbourg(FR)**

(74) Representative: **Gillard, Marie-Louise et al**
**Cabinet Beau de Loménie 55, Rue d'Amsterdam**
**F-75008 Paris(FR)**

## (54) Novel phosphonate derivatives of certain nucleosides.

(57) This invention relates to novel phosphonate derivatives of certain nucleoside analogs, to the methods for their preparation and to their use as anti-viral agents.

EP 0 477 454 A1

This invention relates to novel phosphonate derivatives of certain nucleoside analogs, to the methods for their preparation and to their use as anti-viral agents.

More specifically this invention relates to compounds of the formula

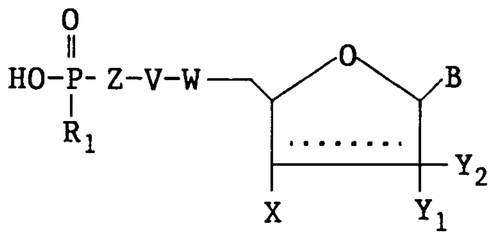

I

the isomeric forms and the pharmaceutically acceptable salts thereof, wherein the dotted line represents a facultative double bond,

$R_1$ is $C_{1-10}$ alkyl, $CH_{3-x}F_x$, $C_{1-10}$ alkyl-OH or $OR_2$, x is zero, 1, 2 or 3,

$R_2$ is H or $C_{1-6}$ alkyl,

Z is O, $CH_2O$, $CF_2$, $CCl_2$, CHF, CHCl, CFCl or is deleted,

V is $(CH_2)_m$ or $(CH_2)_mY-(CH_2)_n$, or is deleted, with m and n being zero, 1, 2, 3 or 4, and Y is -C≡C,

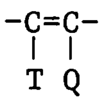 , 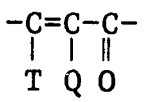 , 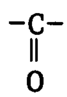 , 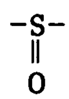 , 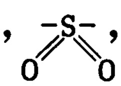 , 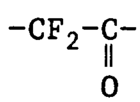 , 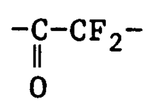 ,

or 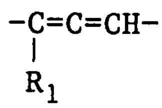 ,

T and Q each are H, F or Cl,

W is O or is deleted, with the provisos that (a) at least one of Z, V or W is other than deleted, (b) the -Z-V-W- moiety is other than -O-, (c) the -Z-V-W- moiety does not contain an oxygen-to-oxygen bond, (d) when X is $N_3$ the -Z-V-W-moiety is other than $-CH_2$ or $-CH_2O$, and (e) when W is oxygen, Z cannot be deleted, B is a base selected from the group

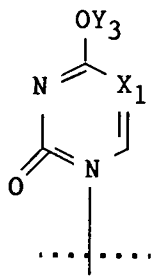

(a)

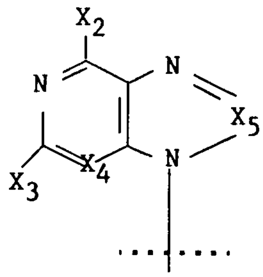

(b)

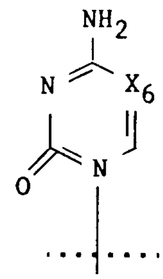

(c)

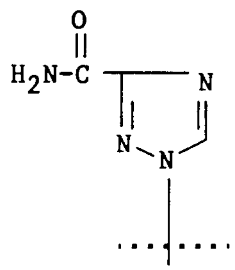

and

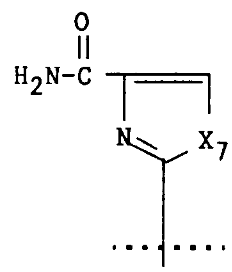

(d) (e)

| | |
|---|---|
| X | is $N_3$, F, Cl, OH or H, |
| $X_1$ | is N or C-$Y_4$ with $Y_4$ being H, $CH_3$, $C_2H_5$, I, F, Cl, Br, NHR, SR, -C≡CH, $CH=CH_2$, CH=CHBr, $CH_2CH_2Cl$, -$CH_2CH_2F$, or $CH_2C_6H_4OCH_2C_6H_5$ (meta), and R is H, $CH_3$, $C_2H_5$, $CH_2C≡CH$, -$CH_2CH=CH$ or $CH_2CN$, |
| $X_2$ | is H, OH, Cl, $NH_2$, $SCH_3$ or SH, |
| $X_3$ | is H, $NH_2$, F, Cl, Br or I, |
| $X_4$ | is N or CH, |
| $X_5$ | is N, $CY_5$ with $Y_5$ being H, OH, $NH_2$, $NHNH_2$, $CH_3$, Cl, Br, or NHMe, |
| $X_5$ | is $CY_6$ with $Y_6$ being H, F, $CH_3$ or CH=CHBr, |
| $X_7$ | is S or Se, |
| $Y_1$ | is H, Cl, F or OH, |
| $Y_2$ | is H, Cl or F, with the proviso that when the dotted line represents a double bond then X is F, Cl, OH or H, $Y_1$ is H, OH, F or Cl and $Y_2$ is deleted, |
| $Y_3$ | is H or $C_2H_5$, |

The term "pharmaceutically acceptable acid addition salts" is intended to apply to any non-toxic organic or inorganic acid addition salts of the base compounds of formula 1. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic forms, demonstrate higher melting points and an increased stability.

Pharmaceutically acceptable metal and amine salts are those salts which are stable under ambient conditions, and wherein the cation does not contribute significantly to the biological activity of the salt. Suitable metal salts include the sodium, potassium, calcium, barium, zinc, and aluminium salts. The sodium and potassium salts are preferred. Suitable amine salts are prepared from amines which have sufficient

basicity to form a stable salt, and preferably include those amines which are frequently used in medicinal chemistry because of their low toxicity and acceptability for medical use. These include the trialkylamines such as triethylamine, and others including procaine, dibenzylamine. N-benzyl-betaphenethylamine, ephenamine, and N,N'-dibenzylethylenediamine, dehydroabietyl-amine, N-ethylpiperidine, benzylamine, and dicyclohexylamine.

The compounds of formula I are depicted in a non-specific representation with respect to stereochemical configuration but it is to be understood that the *cis*-stereochemical form relating to the base and the substituent located at the 4-position of the ribose moiety is preferred.

It is to be noted that the moieties such as

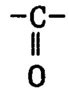 , 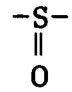 ,

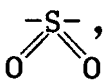 , 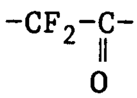 , 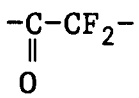 , 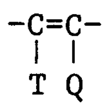 , 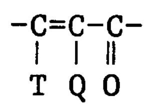 , and 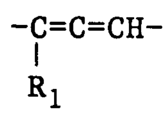 ,

may also be written as C(O), S(O), $S(O)_2$, $CF_2C(O)$, $C(O)CF_2$, C(T) = C(Q), and C(T) = C(Q)C(O) and $-C(R_1)-=C=CH$, respectively, and the

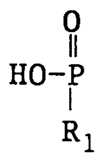

moiety may be written as

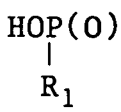

Quite obviously from the fact that Y may be

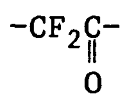 ,

or

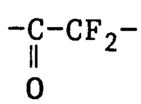 ,

it is important that the defined moieties of -Z-V-W-be read as shown. For example, if Z and W are deleted, and V is $(CH_2)_mY-(CH_2)_n$ with m and n being I with Y being $-CF_2C(O)-$, the

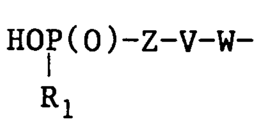

moiety would be

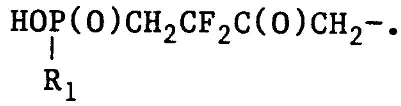

Stated another way if Z and W were deleted, and V was $CH_2C(T)=C(Q)C(O)CH_2$ the correct alignment would be

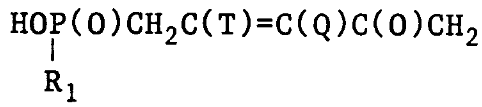

whereas

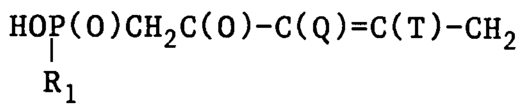

would be an incorrect interpretation.

Representative of the most preferred moieties of the depicted bases of formulae (a), (b), (c), (d) and (e) for defining compounds of formula I are:
guanine,
adenine,
pyrrolo[2,3-d]pyrimidine
pyrazolo[3,4-d]pyrimidine
8-azapurine
benzoguanine
2-aminopurine
8-hydroxy-purine, adenine or guanine
8-methyl-purine, adenine or guanine
8-chloro-purine, adenine or guanine
8-bromo-purine, adenine or guanine
8-iodo-purine, adenine or guanine
8-amino-purine, adenine or guanine
8-methylamino-purine, adenine or guanine
8-dimethylamino-purine, adenine or guanine
5-fluorouracil
5-methyluracil
5-hydroxyuracil
5-bromouracil
5-iodouracil
5-chlorouracil
5-nitrouracil
5-(2-bromovinyl)uracil
5-hydroxymethyluracil
5-fluoro-cytosine
5-bromo-cytosine
5-iodo-cytosine
5-nitro-cytosine
2-amino-6-chloropurine
5-fluoro-4-(methylthio)pyrimidin-2(IH)one
5-bromo-4-(methylthio)pyrimidin-2(IH)one
5-iodo-4-(methylthio)pyrimidin-2(IH)one
5-aza-cytosine

2,6-diaminopurine
xanthine
hypoxanthine

In practice, it is preferred to employ bases having either a pyrimidine or a purine core structure nucleus (including their lactam, lactim and/or tautomeric forms). Preferred pyrimidine type bases are uracil, thymine, cytosine, 5-halogenated (F, Br or I) or 5-carboxy uracils, and 5-methyl or 5-hydroxymethyl-cytosines. Preferred purine type bases are adenine, guanine, 6-methylamino or 6,6-dimethylamino purines or 6-OH purines, 8-azaguanine, and 6-oxy or 2,6-dioxy purines. Otherwise stated, preferred compounds of this invention are those which contain such nucleosidic moieties as adenosine, deoxyadenosine, guanosine, deoxyguanosine, cytidine, deoxycytidine, uridine, deoxyuridine, thymidine, inosine and xanthosine.

In general, the compounds of this invention may be prepared by the application of analogous chemical reactions known in the art using reactants which are already known or which may be prepared using standard processes and techniques well known in the art.

Typically the preparation of the desired compounds of formula I may be effected by a condensation of an activated nucleophile with an electrophile bearing a suitable leaving group. Of course in those instances wherein there are reactive groups on the reactants which would interfere with the efficiency of the reaction such reactive groups may be suitably protected by techniques well known in the art. Further, it is also obvious that, depending on the economics of any particular set of reactants it may be convenient to effect separation of any isomeric forms resulting from any particular step; the separation being effected by standard techniques (e.g. HPLC) well known in the art.

In essence the particular set of reactants utilized for the condensation reactions will depend, to a large degree, upon whether the "W" moiety is oxygen or is deleted. In those instances wherein W is oxygen it is preferred to utilize reactants wherein the activated nucleophile bears the nucleoside-containing moiety, i.e.

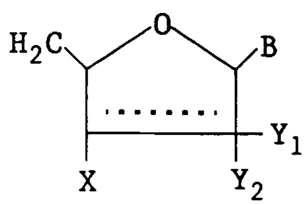

which, for convenience, will be written as $H_2C$-Nu, whilst the electrophile bearing the suitable leaving group will bear the phosphonate moiety. When W is deleted the converse would be true.

The foregoing condensation reactions may be illustrated by the following generically depicted reaction schemes.

**REACTION SCHEME A**

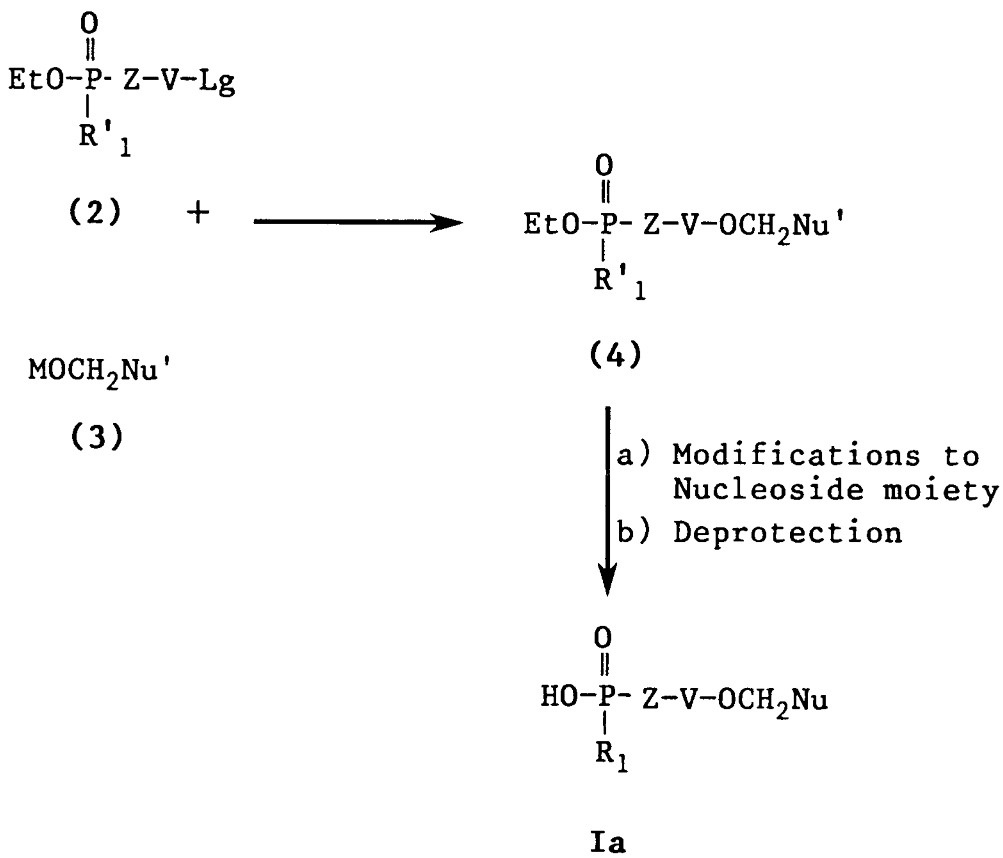


(2) (3) (4) Ia

wherein Et is ethyl, Lg represents a suitable leaving group, M is a metallo cation, $R_1$, Z, V, $CH_2Nu$ are as previously defined, $CH_2Nu'$ is a reaction-protected analog of $CH_2Nu$, $R'_1$ is $-C_{1-10}$ alkyl, $-CH_{3-x}F_x$, x being zero, 1, 2 or 3, $-C_{1-10}$ alkyl-O-$C_{1-6}$ alkyl, or $C_{1-10}$ alkoxy.

Suitable leaving groups for the foregoing reaction include such groups as triflates, alkyl or aryl sulfonates (e.g. $CF_3SO_2O$, tosylate and mesylate) and halides (preferably bromo, chloro or iodo). Suitable metallo cations are Na, K, Li, Cs, Mg, ZnBr or MgBr, preferably when cesium is utilized the condensation is effected in the presence of a crown ether. In effecting the foregoing reactions, the reactants (2) and (3) are contacted together in a suitable anhydrous solvent (e.g. tetrahydrofuran (THF), dimethylformamide (DMF), ether and the like at temperatures generally known for such type reactions, the range being -78°C (e.g. when a lithio derivative is reacted with a bromide in THF) to about room temperature, the reaction generally being conducted in an inert atmosphere using nitrogen or argon. Once the condensation is effected, modifications to the nucleosidic moiety is completed, if necessary, and then the protecting groups, if any, are removed to obtain the desired compounds of Ia.

Examplary of the Z-V-O moieties to which the foregoing reaction scheme would be applicable are:

those wherein Z is $CH_2O$ and V is as generically defined and W is O, Z is $CH_2O$ and V and W are deleted, Z is $CF_2$, $CCl_2$, CHF, CHCl and V is as generically defined with W being O, and Z deleted and V is as generically defined and W is O.

Of course in those instances wherein V contains a carbonyl moiety which may iterfere with the reaction, e.g. V is

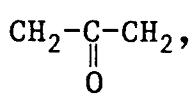

the carbonyl is reduced to its alcohol, the alcohol protected with a trimethylsilyl ether, and following the condensation the silyl ether is cleaved to its alcohol and the alcohol is oxidized (preferably via a Swern oxidation) to the desired ketone.

A variation of the foregoing process is the process depicted by the following generic reaction scheme

**REACTION SCHEME B**

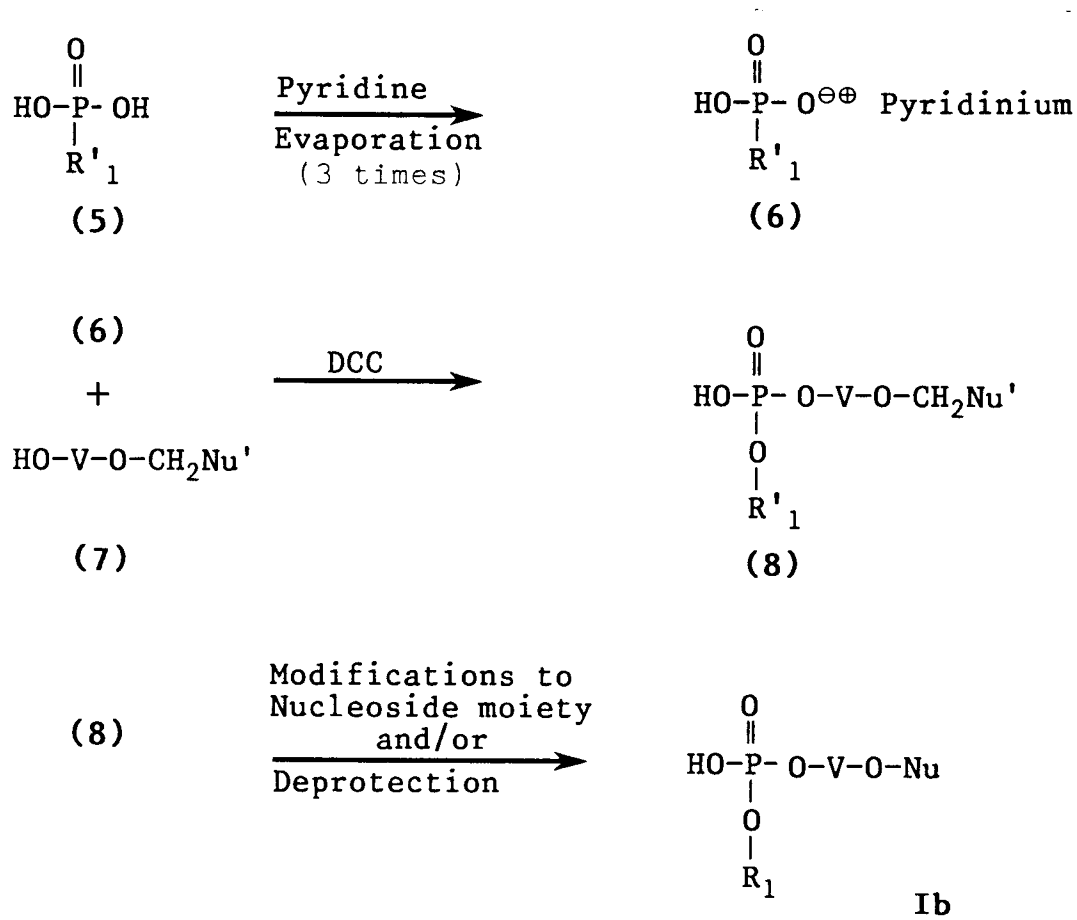


(5) (6)

(6) + (7) (8)

(8) Ib

wherein V, $R_1$, the $-CH_2Nu'$ and $-CH_2Nu$ moieties being as previously defined, and $R'_1$ is a reaction-protected analog of the $R_1$ moiety.

The foregoing reaction is particularly well suited to those compounds of formula I wherein Z and W are both oxygen and V is as generically defined in formula I. In effecting this condensation the phosphonate salt (6) is coupled with the appropriate nucleoside (7) by interaction at about 20°C to 40°C for one to three days in the presence of 3 to 5 equivalents of decyclohexylcarbodiimide (DCC) under anhydrous conditions, preferably in an inert atmosphere (nitrogen or argon being preferred). After the reaction is completed it is quenched with water, the N,N-dicyclohexylurea is filtered, the filtrate washed with diethyl ether and the products are isolated and purified using recrystalization or high performance liquid chromatography (HPLC) techniques. With HPLC, it is preferred to use a reverse phase on a strong anionic column.

In those instances wherein the nucleophilic alkylation process produced a carbon-carbon bond at the site of reaction, the following procedure may be utilized.

**REACTION SCHEME C**

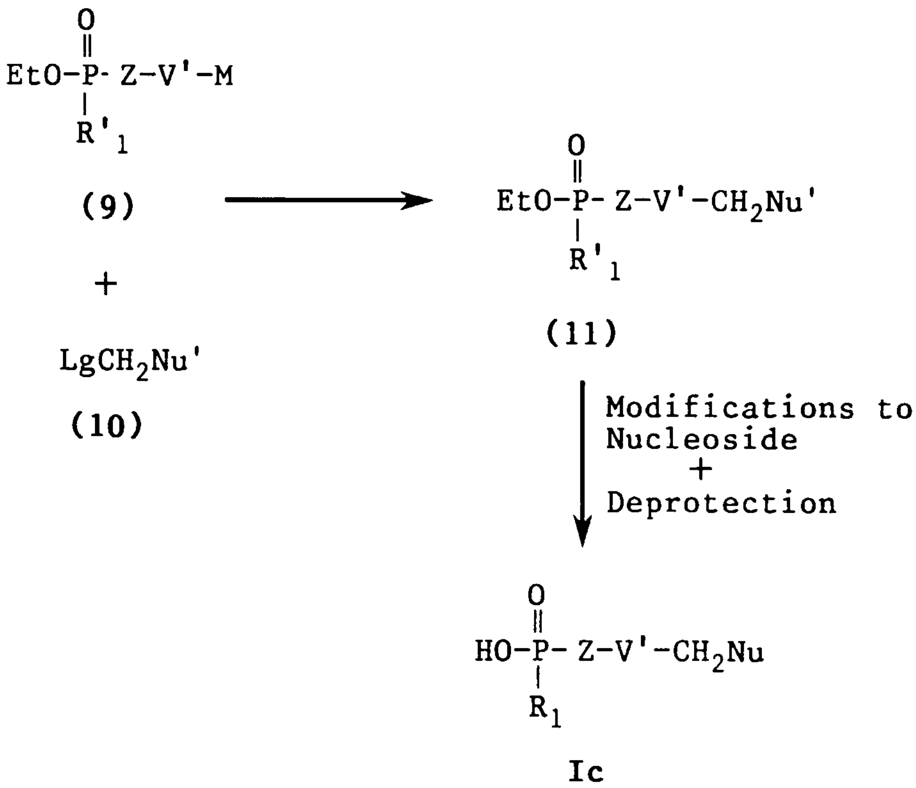


(9) + (10) → (11) → Ic

wherein

| | |
|---|---|
| V' | is as defined except it cannot be deleted or is a reaction-protected analog thereof, and |
| Z, $R'_1$, Et, Lg, Nu and Nu' | are as previously defined. |

Exemplary of the Z-V' moieties to which the foregoing reaction scheme would be applicable are:

O-V'- wherein V' is (other than deleted) as previously defined; $-CH_2O-V'-$; $CF_2$, $CCl_2$, CHF or CHCl-V; $CF_2$; $CCl_2$; CHF; CHCl; and V'.

Of course, in certain instances the process of Reaction Scheme C is modified to accomodate the known properties of the reactants. For example, to prepare a compound of Formula I wherein the Z-V-W moiety is CHF it is preferred to prepare a lithio derivative of a tetraethyl fluoromethylene bisphosphonate which is reacted with an aldehyde derivative of the $CH_2Nu'$

(i.e., 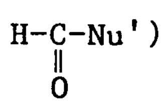),

and the resulting olefin of the condensation reaction is chemically reduced and the resulting product deprotected to the desired product. Alternatively an ester derivative of the $CH_2Nu$

(i.e., 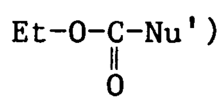),

moiety may also be utilized in a condensation reaction with a metallo derivative of the phosphonate (9) and the resulting product is chemically reduced, the product of which is then deprotected to produce the desired compounds of Ic.

Of course, in those instances of Reaction Scheme C wherein it is desired, homologation procedures well known in the art may be employed to elongate the alkylene moiety of the 5'-nucleoside (i.e., $CH_2Nu$). A

suitable process may be illustrated as:

$HOCH_2Nu \rightarrow CNCH_2Nu \rightarrow HO(CH_2)_2Nu \rightarrow CN(CH_2)_2Nu \rightarrow HO(CH_2)_3Nu$ and so on.

The following specific examples will serve to adequately illustrate the types of processes generically described above but such examples are not intended to be limiting; it being obvious to one of ordinary skill in the art that such may be adapted to suit any given pair of reactants for the preparation of the compounds of this invention.

## EXAMPLE 1

### 5'-O-[3-O-(Trifluoromethylphosphinico)-3-hydroxy-2-oxopropyl]-3'-fluoro-2',3'-dideoxy-5-chlorouridine

Step A:

To a stirred solution of 3'-fluoro-2',3'-dideoxy-5'-chlorouridine (1.05 g, 4 mmol) in dimethyl sulfoxide (20 ml), sodium hydride (0.17 g, 8 mmol) is added. The stirring is continued at room temperature under nitrogen and the mixture is treated with α'-tetrahydropyranyloxy-α-bromoacetone (0.96 g, 4 mmol). The stirring is continued for 24 hours at room temperature. The mixture is neutralized with acetic acid and concentrated *in vacuo*. The residue is diluted with ethanol and p-toluenesulfonic acid (0.1 g) is added and stirred overnight at room temperature. The mixture is concentrated and purified by flash chromatography on silica gel with ethyl acetate: ethanol, 95:5 as an eluent to yield 5'-O-(3-hydroxy-2-oxopropyl)-3'-fluoro-2',3'-dideoxy-5-chlorouridine. Yield: 0.9 g, 75%.

Step B:

5'-O-(3-hydroxy-2-oxopropyl)-3'-fluoro-2',3'-dideoxy-5-chlorouridine (0.31 g, 1 mmol) and trifluoromethyl phosphonic acid (0.3 g, 2 mmol) are dissolved in anhydrous pyridine (10 ml) and the solvent is removed in partial vacuum at 35°C. This procedure is repeated twice to provide an anhydrous reaction mixture. The residue is dissolved in anhydrous pyridine (20 ml) and dicyclohexylcarbodiimide (0.82 g, 4 mmol) is added. After stirring at 35°C under nitrogen atmosphere for 20 hours, water (10 ml) is added, dicyclohexyl urea is filtered off and the pH of the filtrate is adjusted to 7 with 1M ammonium hydroxyde. The product is purified by ion exchange chromatography (DOWEX AG 1X4), eluted with 4N formic acid to yield 5'-O-(3-O-(trifluoromethylphosphinico)-3-hydroxy-2-oxopropyl]-3'-fluoro-2',3'-dideoxy-5-chlorouridine. Yield: 0.25 g, 55%.

## EXAMPLE 2

### 5'-O-[4-O-(-n-Butylphosphinico)-4-hydroxy-2-butynyl]-2',3'-dihydro-3'-deoxythymidine

Step A:

To a stirred solution of 2',3'-dihydro-3'-deoxythymidine (0.43 g, 2 mmol) in dimethylsulfoxide (10 ml), sodium hydride (0.85 g, 4 mmol) is added, the stirring continued at room temperature under nitrogen and the mixture is treated with 1-bromo-4-tetrahydropyranyloxy-2-butyne (0.47 g, 2 mmol). The stirring is continued for 24 hours at room temperature. The mixture is neutralized with acetic acid and concentrated *in vacuo*. The residue is taken up in ethanol and p-toluene sulfonic acid is added (0.05 g) and stirred overnight at room temperature. The mixture is then concentrated and purified by flash chromatography on silica gel with ethyl acetate: ethanol, 95:5 as an eluent to yield 5'-O-(4-hydroxy-2-butynyl)-2',3'-dihydro-3'-deoxythymidine. Yield: 0.15 g, 60%.

Step B:

5'-O-(4-hydroxy-2-butynyl)-2',3'-dihydro-3'-deoxythymidine (0.13 g, 0.5 mmol) and n-butylphosphonic acid (0.14 g, 1 mmol) are dissolved in anhydrous pyridine (5 ml) and the solvent is removed in partial vacuum at 35°C. The residue is dissolved in anhydrous pyridine (10 ml) and dicyclohexylcarbodiimide (0.4 g, 2 mmol) is added. After stirring at 35°C under nitrogen atmosphere for 20 hours, water (10 ml) is added

and dicyclohexylurea is filtered off. Then the pH of the mixture is adjusted to 7 with 1M ammonium hydroxyde and the product is purified by ion exchange chromatography (DOWEX AG 1×4), eluted with 4N formic acid to yield 5'-O-[4-O-(-n-butylphosphinico)-4-hydroxy-2-butynyl]-2',3'-dihydro-3'-deoxythymidine. Yield: 0.11 g, 50%.

**EXAMPLE 3**

**5'-O-[3-O-(Methylphosphinico)-3-hydroxypropyl]-3'-azido-3'-deoxythymidine**

Step A:

To a stirred solution of 3'-azido-3'-deoxythymidine (1.25 g, 5 mmol) in dimethylsulfoxide (30 ml), sodium hydride (0.2 g, 10 mmol) is added, the stirring is continued at room temperature under nitrogen and the mixture is treated with 3'-tetrahydropyranyloxy-1-iodopropane (1.35 g, 5 mol). The stirring is continued for 24 hours at room temperature. The mixture is neutralized with acetic acid and concentrated *in vacuo*. The residue is diluted with ethanol and p-toluenesulfonic acid (0.1 g) is added and stirred overnight at room temperature. The mixture is concentrated and purified by flash chromatography on silica gel with ethylacetate: ethanol, 95:5 as an eluent to yield 5'-O-(3-hydroxypropyl)-3'-azido-3'-deoxythymidine. Yield: 1.25, 65%.

Step B:

A solution of dicyclohexylcarbodiimide (2.5 g, 12 mmol) in anhydrous pyridine (5 ml) is added to a solution of 5'-O-(3-hydroxypropyl)-3'-azido-3'-deoxythymidine (0.92 g, 3 mmol) and methylphosphonic acid (0.58 g, 6 mmol) in anhydrous pyridine (10 ml). After stirring at 35°C under nitrogen atmosphere for 20 hours, water (10 ml) is added, dicyclohexylurea is filtered off and the pH of the filtrate is adjusted to 7 with 1M ammonium hydroxyde. The product is purified by ion exchange chromatography (DOWEX AG 1X4), eluted with 4N formic acid to yield 5'-O-[3-O-(methylphosphinico)-3-hydroxypropyl]-3'-azido-3'-deoxythymidine. Yield: 0.65 g, 54%.

**EXAMPLE 4**

Step A:

5'-O-(Diethoxyphosphorylmethyl)-2',3'-dideoxy-didehydrothymidine

50 mmol of sodium hydride (1.5 g of a 80% suspension in oil) are added portionwise to a stirred solution of 22.3 mmol (5 g) of 2',3'-dideoxy-didehydrothymidine (prepared as described in J. Med. Chem. 32, 461, 1989) in 20 ml of anhydrous dimethylformamide at 20°C under an argon atmosphere. The reaction mixture is stirred at 20°C for 1 hour and 7.4 g (23 mmol) of diethyl p-toluenesulfonyloxymethyl phosphonic acid diethyl ester dissolved in 10 ml of anhydrous dimethylformamide are added to the reaction mixture which is stirred at 20°C for two days. The reaction mixture is evaporated to dryness under reduced pressure. The residue is directly purified by flash chromatography on silica gel (chloroform, ethanol) to give 10 mmol (3.7 g) of the expected product (43%).

Step B:

5'-O-(Phosphonomethyl)thymidine-2',3'-dideoxy-didehydrothymidine

40 mmol (6.12 g) of trimethylsilylbromide are slowly added to a stirred suspension of 5'-O-(diethoxyphosphorylmethyl)-2',3'-dideoxy-didehydrothymidine (10 mmol, 3.7 g) in 40 ml of anhydrous dimethylformamide at 20°C under argon. The reaction mixture is stirred at 35°C for 5 hours. Dimethylformamide is evapored by distillation under reduced pressure. The residue is dissolved in anhydrous acetonitrile (35 ml) and the final product is obtained on crystallization by addition of water (0.75 ml). The white crystals are collected by filtration and dried at 60°C under vacuum, giving 2.5 mmol (800 mg) of the expected product (25%).

**EXAMPLE 5**

Step A:

1,1-Difluoro-4-bromobutylphosphonic acid diethyl ester

50 mmol of difluoromethylphosphonic acid diethyl ether dissolved in 50 ml of anhydrous tetrahydrofuran are slowly added to a solution of 50 mmol of lithium diisopropylamine in 5 ml of tetrahydrofuran at -78°C under argon. The reaction mixture is stirred for 45 minutes at -78°C and a cooled (-78°C) solution of 1,3-dibromopropane (100 mmol) in 50 ml of tetrahydrofuran is rapidly (5 minutes) added via a syringe. The reaction mixture is stirred at -78°C for 3 hours, at -20°C for 2 hours, and quenched by addition of 50 ml of saturated aqueous ammonium chloride. Tetrahydrofuran is removed by evaporation under reduced pressure and the residue is extracted three times with 150 ml portions of ethyl acetate. The organic layers are combined, washed with 40 ml of brine, dried over sodium sulfate, filtered and evaporated to give 26 g of a crude oil which is purified by flash chromatography on silica gel giving 24 mmol (7.4 g) of the expected product (48%).

Step B:

5'-O[4-(Diethoxyphosphoryl)-4,4-difluorobutyl]-2',3'-dideoxyinosine

70 mmol of sodium hydride (2.1 g of a 80% suspension in oil) are added portionwise to a stirred suspension of 35 mmol of 2',3'-dideoxyinosine in 35 ml of anhydrous dimethylformamide at 0°C under argon. After 2 hours at -78°C, 24 mmol of 1,1-difluoro-4-bromobutylphosphonic acid diethyl ester dissolved in 10 ml of dimethylformamide are slowly added to the reaction mixture which is stirred at 20°C for 2 days. Dimethylformamide is evaporated under reduced pressure to give 17 g of a residue which is suspended in 25 ml of aqueous saturated ammonium chloride and extracted five times with 60 ml portions of ethyl acetate. The organic layers are dried over sodium sulfate, filtered and evaporated to give 7 g of a residue which is purified by flash chromatography on silica gel (chloroform/ethanol) to give 3.89 g of the expected product (35% yield).

Step C:

5'-O-(4-Phosphono-4,4-difluorobutyl)-2',3'-dideoxyinosine

The final product is obtained by the method described in Example 4, Step B.

**EXAMPLE 6**

Step A:

5'-(Diethoxyphosphoryl-fluoromethylene)-3'-fluoro-2',3',5'-trideoxy-5-chlorouridine

A solution of 3'-fluoro-2',3'-dideoxy-5-chlorouridine (15 mmol, 3.96 g) and dicyclohexylcarbodiimide (9.38 g, 45 mmol) in dimethylsulfoxide (68 ml) was treated with pyridine (1.3 ml, 15 mmol) followed by trifluoroacetic acid (0.6 ml, 3.3 mmol) and the reaction mixture was stirred for 24 hours at room temperature. After removal of the precipitated dicyclohexylurea by filtration, the filtrate was slowly added to a stirred solution of 35 mmol of tetraethyllithiofluoromethylene bisphosphonate dissolved in 50 ml of anhydrous tetrahydrofuran at -78°C under argon. The reaction mixture was stirred at -78°C for 1 hour and at 20°C for 20 hours before it was evaporated to dryness *in vacuo* and directly purified by flash chromatography on silica gel using chloroform and increasing concentrations of ethanol as eluent to give 6.5 mmol of the expected product (2.7 g, 43%).

Step B:

5'-(Diethoxyphosphoryl-fluoromethyl)-3'-fluoro-2',3',5'-trideoxy-5-chlorouridine

To a mixture of the olefin (2.7 g, 6.5 mmol) and potassium azodicarboxylate (6.6 g, 34 mmol) in pyridine (60 ml) was added dropwise with stirring under an argon atmosphere a solution of 2.92 ml (50.8 mmol) of acetic acid in 14 ml of pyridine. The mixture was stirred for 24 hours before it was filtered into 1 L of water,

which was extracted 5 times with 50 ml portions of chloroform. Evaporation of the dried chloroform extract gave 2.1 g of partially reduced material. A solution of this material in ethanol (40 ml) containing 400 mg of 5% palladium on barium sulfate catalyst was reduced overnight at atmosphere pressure. The catalyst was removed by filtration and the filtrate evaporated to dryness *in vacuo* to give a yellow oil which was purified by flash chromatography on silica gel (chloroform and increasing concentrations of ethanol as eluents) to give 4.5 mmol of the expected product 5'-(diethoxyphosphoryl-fluoromethyl)-3'-fluoro-2',3',5'-trideoxy-5-chlorouridine (1.88 g) 70% yield.

Step C:

5'-(Phosphonofluoromethyl)-3'-fluoro-2',3',5'-trideoxy-5-chlorouridine

The final product is obtained by the method described for the preparation of Example 4, Step B.

**EXAMPLE 7**

Step A:

Synthesis of diethyl(2-trifluoromethanesulfonyloxyethoxy)-methane phosphonate

13.4 ml (80 mmol) of triflic anhydride are slowly added to a solution of pyridine (85 mmol, 6.70 g) in 150 ml of anhydrous dichloromethane at -10 ° C under argon. The white suspension is stirred at -10 ° C for 20 minutes and 40 mmol (8.48 g) of 2-hydroxyethoxymethane phosphonic acid diethyl ester dissolved in 40 ml of dichloromethane are slowly added to the reaction mixture at -20 ° C. After stirring during 2 hours at -20 ° C and 1 hour at 20 ° C, the reaction mixture is poured into iced water and extracted 3 times with 80 ml portions of dichloromethane. The organic layers are collected, washed with brine, dried over sodium sulfate, filtered and evaporated to give a crude oil which is directly purified by flash chromatography on silica gel affording 20 mmol (10.32 g) of the expected triflate (75% yield).

Step B:

5'-O-[2-O-(Diethoxyphosphorylmethyl)-2-hydroxyethyl]-3'-azido-3'-deoxythymidine

70 mmol of sodium hydride (2.1 g of a 80% solution in oil) are added portionwise to a stirred suspension of 35 mmol of 3'-deoxy-3'-azidothymidine in 35 ml of anhydrous dimethylformamide at 0 ° C under argon. After 2 hours at -78 ° C 30 mmol of the previously described triflate derivative dissolved in 10 ml of dimethylformamide are slowly added to the reaction mixture which is stirred at 20 ° C for 40 hours. Dimethylformamide is evaporated under reduced pressure to give 16 g of a residue which is suspended in 25 ml of saturated aqueous ammonium chloride and extracted five times with 60 ml portions of ethyl acetate. The organic layers are combined, dried over sodium sulfate, filtered and evaporated to give 9 g of a crude product which is purified by flash chromatography on silica gel (chloroform, ethanol) to give 6.7 g of the expected product (42% yield).

Step C:

5'-O-[2-O-(Phosphonomethyl)-2-hydroxyethyl]-3'-azido-3'-deoxythymidine

The final product is obtained by the method described for the preparation of Example 4, Step C.

**EXAMPLE 8**

**5'-(Phosphonodifluoromethyl)-3'-azido-5',3'-dideoxythymidine**

Step A:

5'-Trifluoromethanesulfonyl-3'-azido-3'-deoxythymidine

Triflic anhydride (6.7 ml, 40 mmol) is slowly added under an atmosphere of nitrogen to a cooled (-10°C) and stirred solution of pyridine (3.5 ml, 44 mmol) in 120 ml of dichloromethane. The white suspension is stirred for 10 minutes at -10°C and then cooled to -20°C, when a solution of 3'-azido-2',3'-dideoxythymidine (4.58 g, 30 mmol) in anhydrous tetrahydrofuran is slowly added. Stirring is continued for 2 hours at -20°C and 1 hour at room temperature. Crushed ice is added, phases separated, and the aqueous layer extracted with dichloromethane (2 × 50 ml). The combined organic phases are washed with brine, dried (magnesium sulfate) and evaporated (20 Torr, 30°C) to afford an oil. Flash chromatography on silica gel (230-400 mesh, eluent ethyl acetate:petroleum ether 1:1) and evaporation of the product-containing fractions gives 9.6 g (80%) of the title compound as an oil.

Step B:

5'Diethoxyphosphoryldifluoromethyl)-3'-azido-5',3'-dideoxythymidine

A solution of difluoromethylphosphoric acid diethyl ester (9.4 g, 50 mmol) in 50 ml of anhydrous tetrahydrofuran is added slowly under an atmosphere of nitrogen to a cooled (-78°C) and stirred solution of lithiumdiisopropylamide (prepared from 52 mmol butyllithium and 52 mmol diisopropylamine) in anhydrous tetrahydrofuran (50 ml). The reaction mixture is stirred for 45 minutes at -78°C and the precooled (-78°C) solution of the above-prepared triflate (9.6 g, 24 mmol) in 50 ml of anhydrous tetrahydrofuran is rapidly added via a syringe. Stirring is continued for 16 hours at -78°C. A saturated solution of ammonium chloride is added and the mixture allowed to warm up to room temperature. Solvents are evaporated to half of the original volume (20 Torr, 30°C) and the residue is exhaustively extracted with ethyl acetate (3 × 150 ml). The combined organic layers are washed with brine, dried over magnesium sulfate, and evaporated (20 Torr, 30°c) to give an oil which is purified by flash chromatography on silica gel (230 mesh, eluent ethyl acetate). The combined product-containing fractions are evaporated (20 Torr, 30°C, then 0.01 Torr, 20°C) to afford 1.57 g (15%) of the title compound as an oil.

Step C:

5'-(Phosphonodifluoromethyl)-3'-azido-5',3'-dideoxythymidine

At room temperature, trimethylsilylbromide (2.2 g, 14.4 mmol) is added slowly under nitrogen to a stirred suspension of the ester of 5'diethoxyphosphoryldifluoromethyl)-3'-azido-5',3'-dideoxythymidine (1.57 g, 3.6 mmol) in 15 ml of anhydrous dimethylformamide. Stirring is continued at 35°C for 35 hours. The solvent is removed by evaporation under reduced pressure (0.01 Torr, 20-30°C) and the residue dissolved in anhydrous acetonitrile (10 ml). Precipitation of the title compound is caused by addition of water (0.25 ml). Filtration of the mixture and drying of the filter residue (60°C, 0.01 Torr) affords 343 mg (25%) of the final compound.

**EXAMPLE 9**

**5'-O-(4-Phosphono-2-oxobutyl)-3'-fluoro-2',3'-dideoxy-5-chlorouridine**

Step A:

5'-O-[4-Diethoxyphosphoryl-2-(tert.butyldimethylsilyloxy)-butyl]-3'-fluoro-2',3'-dideoxy-5-chlorouridine

Sodium hydride (100 mmol, 3.0 g of a 80% suspension in oil) is added portionwise to a stirred solution of 3'-fluoro-2',3'-dideoxy-5-chlorouridine (13.2 g, 50 mmol) in 40 ml of anhydrous dimethylformamide. The reaction mixture is stirred under an atmosphere of nitrogen for 1 hour. A solution of 2-tert.butyldimethylsilyloxy-4-bromobutyl phosphonic acid diethyl ester (20.2 g, 50 mmol) in 20 ml dimethylformamide is added and the resulting mixture stirred at room temperature for two days. Evaporation of the solvent (0.01 Torr, 20-30°C) and flash chromatography of the oily residue on silica gel (200-400 mesh, eluent chloroform/ ethanol) affords, after evaporation of the pooled product-containing fractions (20 Torr, then 0.1 Torr) 23.4 g (40%) of the desired phosphonate.

Step B:

5'-[4-(Diethoxyphosphoryl)-2-hydroxybutyl]-3'-fluoro-2',3'-dideoxy-5-chlorouridine

A 1M solution of tetrabutylammonium fluoride in tetrahydrofuran (40 mmol) is added to a stirred solution of 5'-O-[4-diethoxyphosphoryl-2-(tert.butyldimethylsilyloxy)-butyl]-3'-fluoro-2',3'-dideoxy-5-chlorouridine (23.4 g, 20 mmol) in tetrahydrofuran (100 ml). Stirring of the reaction mixture is continued for 6 hours at room temperature. Then tetrahydrofuran is evaporated and the oily residue applied to flash chromatography on silica gel (230-400 mesh, eluent chloroform/ethanol). Evaporation of the pooled product containing fractions afforded 6.6 g (70%) of the desired alcohol.

Step C:

5'-O-(4-Diethoxyphosphoryl-2-oxobutyl)-3'-fluoro-2',3'-dideoxy-5-chlorouridine

To a cooled (-55°C) and stirred solution of oxalylchloride (2.54 g, 20 mmol) in anhydrous dichloromethane (20 ml) is added under nitrogen a solution of 3.43 g anhydrous dimethylsulfoxide (44 mmol) in dichloromethane (20 ml) at such a rate that the reaction temperature does not exceed -50°C (5minutes). Stirring is continued for 15 minutes at -55°C and a solution of 5'-[4-(diethoxyphosphoryl)-2-hydroxybutyl]-3'-fluoro-2',3'-dideoxy-5-chlorouridine (6.6 g, 14 mmol) in dichloromethane/dimethylsulfoxide (20 ml/l ml) is added over a period of 2 minutes. The reaction mixture is then allowed to warm up to -30°C for 5 minutes and cooled again to -55°C. After 15 minutes stirring at -55°C triethylamine (10 g, 100 mmol) is added at a rate to keep the reaction temperature below -50°C. The mixture is stirred for 20 minutes at -55°C and a saturated solution of aqueous citric acid is added. Having reached room temperature the reaction mixture is diluted with dichloromethane (50 ml), the aqueous layer extracted with dichloromethane (2 × 50 ml). The combined organic phases are washed with brine. Drying over magnesium sulfate and evaporation of the solvents (20 Torr, 30°C) gives an oil which is applied to flash chromatography on silica gel (230-400 mesh, eluent chloroform/ethanol). Evaporation of product-containing fractions affords 1.18 g (60%) of the desired ketone as an oil.

Step D:

5'-O-(4-Phosphono-2-oxobutyl)-3'-fluoro-2',3'-dideoxy-5-chlorouridine

Saponification of the diethyl phosphonate: 5'-O-(4-diethoxyphosphoryl-2-oxobutyl)-3'-fluoro-2',3'-dideoxy-5-chlorouridine (1.18 g, 8.4 mmol) is performed as described for the synthesis of the phosphonate 5-methyl-1-(2',3',4',5',-tetradeoxy-6',6'-difluoro-3'-azido-6'-phosphono-β-D-ribohexofuranosyl)-2,4-(1H,3H)-pyrimidinedione and affords 1.04 g (30%) of the final compound as a semisolid.

**EXAMPLE 10**

**5'-(4-Phosphoryl-4,4-difluoro-1-butynyl)-5',3',2'-trideoxyguanosine**

Step A:

(1,1-Difluoro-4-trimethylsilyl-3-butynyl)phosphonic acid, diethyl ester

A solution of difluoromethyl phosphonic acid diethyl ester (4.7 g, 25 mmol) in anhydrous tetrahydrofuran (25 ml) is added slowly under an atmosphere of nitrogen to a cooled (-78°C) and well stirred solution of lithiumdiisopropylamide (25 mmol) in 50 ml of anhydrous tetrahydrofuran. The reaction mixture is stirred for 45 minutes at -78°C and a precooled (-78°C) solution of 3-trimethylsilyl-1-bromo-2-propyne (4.4 g, 23 mmol) in tetrahydrofuran (25 ml) is added via a syringe. The resulting mixture is then stirred for three hours at -78°C and two hours at -20°C. A saturated solution of aqueous ammonium chloride (20 ml) is added and the mixture evaporated to $\frac{1}{4}$ of its original volume. The mixture is exhaustively extracted with diethyl ether (3 × 50 ml) and the combined organic extracts washed with brine. Drying over magnesium sulfate and evaporation of solvents gives an oil which is further purified by flash chromatography on silica gel. Evaporation of the product containing fractions affords 2.74 g (40%) of the protected acetylene derivative.

Step B:

(1,1-Difluoro-3-butynyl)phosphonic acid, diethyl ester

A 1M solution of tetrabutylammonium fluoride in tetrahydrofuran (10 mmol) is added to a stirred solution of the above-prepared silyl derivative (1,1-difluoro-4-trimethylsilyl-3-butynylphosphonic acid, diethyl ester), (2.74 g, 9.19 mmol) in tetrahydrofuran (20 ml). Stirring of the reaction mixture is continued for 6 hours at room temperature. Then tetrahydrofuran is evaporated and the oily residue purified by Kugelrohr distillation ($Kp_{0.1}$ : 70-90° C - oven temperature) to afford 1.23 g (60%) of the desired acetylene.

Step C:

5'-[4-(Diethoxyphosphoryl)-4,4-difluoro-1-butynyl]-5',3',2'-trideoxyguanosine

Triflic anhydride (2.82 g, 10 mmol) is added under an atmosphere of nitrogen via a syringe to a cooled (0° C) and well stirred solution of 2',3'-dideoxyguanosine (1.25 g, 5 mmol) and pyridine (0.78 g, 10 mmol) in 50 ml of anhydrous tetrahydrofuran. Stirring is continued for 1 hour at 0° C. Water (5 ml) is added and the solution evaporated to $\frac{1}{4}$ of its original volume. Ethyl acetate (100 ml) is added and phases separated. The organic layer is dried over magnesium sulfate and the solvents evaporated. The resulting triflate (oil) is used as such in the following reaction.

A solution of (1,1-difluoro-3-butynyl)phosphonic acid, diethyl ester (1.23 g, 5.5 mmol) in 10 ml of anhydrous tetrahydrofuran is added via a syringe to a cooled (-78° C) solution of lithiumdiisopropylamide (8.3 mmol) in 10 ml of anhydrous tetrahydrofuran. Stirring at -78° C is continued for 1 hour. Then a solution of the above-prepared triflate in 10 ml of tetrahydrofuran is added slowly via a syringe. Stirring is continued for 30 minutes at -78° C and 30 minutes at -20° C. A saturated solution of ammonium chloride is added and the reaction mixture allowed to warm to room temperature. Solvents are evaporated and the oily residue applied to flash chromatography on silica gel (230-400 mesh, eluent chloroform/ethanol: 13/1). Product-containing fractions are evaporated (20 Torr, 30° C, then 0.01 Torr, 30° C) to afford 1.0 g (40%) of the expected dideoxyguanosine.

Step D:

5'-[4-(Ethoxyphosphinico)-4,4-difluoro-1-butynyl]-5',3',2'-trideoxyguanosine

A 1M solution of aqueous sodium hydroxide (4 ml) is added to a stirred solution of the phosphonodiester [5-[(2-amino-1,6-dihydro-6-oxo-9H-purin-9-yl)-2,5-dihydro-2-furanyl]-1,1-difluoro-3-pentynyl]phosphonic acid, diethyl ester (1.0 g, 2.2 mmol) in 5 ml of ethanol. The reaction mixture is stirred for 16 hours at room temperature. The a saturated solution of citric acid is added to achieve a neutral pH. All solvents are evaporated under reduced pressure and the residual solid treated with hot (~50° C) tetrahydrofuran. The solution is then washed with cold (~0° C) brine and dried (magnesium sulfate). Evaporation affords 380 mg (40%) of the final monoester as an oil.

The compounds of this invention (I) have utility as antiviral, antimicrobial, and antitumor agents including tumors in experimental animals. Their antiviral properties can be measured and demonstrated by any of a variety of laboratory methods which are available for this purpose. Similarly, utility against plant pathogens and microbial infections caused by bacteria and other microorganisms can be demonstrated by methods which are well known to those skilled in the art. Their antitumor activity can be shown by established techniques in animals bearing experimental tumors or *in vitro* by using various animal and human tumors. The particular spectrum of viruses and microorganisms against which the present substances are active varies from compound to compound. The murine leukemia virus is an example of a retrovirus which is sensitive to the present compounds. A number of disease conditions including AIDS are caused by retroviruses.

Pharmaceutical compositions, both veterinary and human, containing the claimed compounds which are appropriate for antiviral use are prepared by methods and contain excipients which are well known in the art. A generally recognized compendium of such methods and ingredients is Remington's Pharmaceutical Sciences by E. W. Martin, (Mark Publ. Co., $15^{th}$ Ed., 1975).

The compounds of the invention may be administered parenterally (for example, by intravenous, subcutaneous, intraperitoneal, or intramuscular injection), orally, topically, intranasally, or rectally.

The compositions are administered orally or parenterally at dose levels of about 0.1 to 300 mg/kg of compound of Formula I, preferably 1.0 to 30 mg/kg of body weight. Unit dosage forms administered one to five times daily in the amount of 10 to 500 mg per unit dose, are contemplated for man.

For parenteral administration or for administration as drops, as for eye infections, the compounds may be presented in aqueous solution in a concentration of from about 0.1 to 10%, more preferably about 0.1 to

7%. The solution may contain antioxidants, buffers, and other suitable additives.

Alternatively for infections of the eye, or other external tissues, e.g. mouth and skin, the compositions are preferably applied to the infected part of the body of the patient topically as an iontment, cream, aerosol or powder, preferably as an ointment or cream. the compounds may be presented in an ointment, for instance with a water soluble ointment base, or in a cream, for instance with an oil in water cream base, in a concentration of from about 0.01 to 10%.

The compounds of the present invention or compositions containing them are also useful in treating non-human mammals, bird, e.g., chickens and turkeys, and cold-blooded animals, e.g., fish.

Fish which are in a confined area such as a pool, aquarium or holding tank may also be treated for viral infections such as herpes-like viruses, e.g., channel catfish virus (CCV), herpes-virus salomones, Nerka virus and the like by adding the compound directly to the water of the pool, aquarium, or holding tank or by incorporating the compounds into the feed.

The exact regimen for administration of the compounds and compositions disclosed herein will necessarily be dependent upon the needs of the individual subject being treated, the type of treatment and, of course, the judgement of the attending physician.

## Claims

1. Compounds of the formula

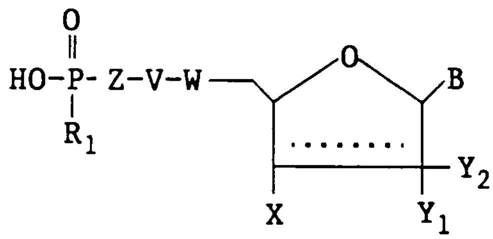

I

the isomeric forms and the pharmaceutically acceptable salts thereof, wherein the dotted line represents a facultative double bond,

- $R_1$ is $C_{1-10}$ alkyl, $CH_{3-x}F_x$, $C_{1-10}$ alkyl-OH or $OR_2$, x is zero, 1, 2 or 3,
- $R_2$ is H or $C_{1-6}$ alkyl,
- Z is O, $CH_2O$, $CF_2$, $CCl_2$, CHF, CHCl, CFCl or is deleted,
- V is $(CH_2)_m$ or $(CH_2)_mY-(CH_2)_n$, or is deleted, with m and n being zero, 1, 2, 3 or 4, and Y is -C≡C,

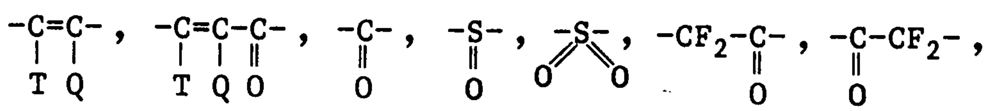

or 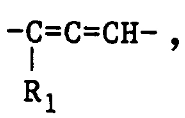 ,

- T and Q each are H, F or Cl,
- W is O or is deleted, with the provisos that (a) at least one of Z, V or W is other than deleted, (b) the -Z-V-W- moiety is other than -O-, (c) the -Z-V-W- moiety does not contain an oxygen-to-oxygen bond, (d) when X is $N_3$ the -Z-V-W-moiety is other than $-CH_2$ or $-CH_2O$, and (e) when W is oxygen, Z cannot be deleted, B is a base selected from the group

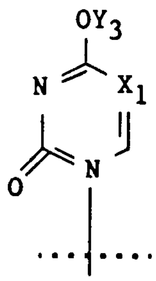

(a)

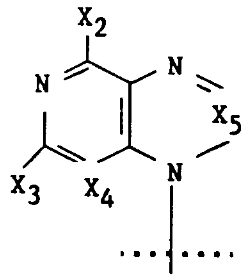

(b)

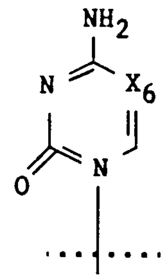

(c)

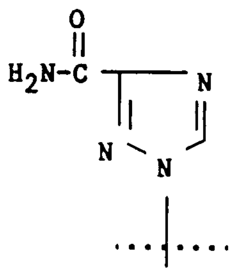

(d)

and

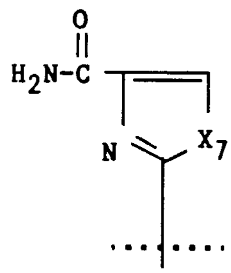

(e)

| | |
|---|---|
| X | is $N_3$, F, Cl, OH or H, |
| $X_1$ | is N or C-$Y_4$ with $Y_4$ being H, $CH_3$, $C_2H_5$, I, F, Cl, Br, NHR, SR, -C≡CH, $CH=CH_2$, $CH=CHBr$, $CH_2CH_2Cl$, -$CH_2CH_2F$, or $CH_2C_6H_4OCH_2C_6H_5$ (meta), and R is H, $CH_3$, $C_2H_5$, $CH_2C≡CH$, -$CH_2CH=CH$ or $CH_2CN$, |
| $X_2$ | is H, OH, Cl, $NH_2$, $SCH_3$ or SH, |
| $X_3$ | is H, $NH_2$, F, Cl, Br or I, |
| $X_4$ | is N or CH, |
| $X_5$ | is N or C$Y_5$ with $Y_5$ being H, OH, $NH_2$, $NHNH_2$, $CH_3$, Cl, Br, or NHMe, |
| $X_5$ | is C$Y_6$ with $Y_6$ being H, F, $CH_3$ or $CH=CHBr$, |
| $X_7$ | is S or Se, |
| $Y_1$ | is H, Cl, F or OH, |
| $Y_2$ | is H, Cl or F, with the proviso that when the dotted line represents a double bond then X is F, Cl, OH or H, $Y_1$ is H, OH, F or Cl and $Y_2$ is deleted, |
| $Y_3$ | is H or $C_2H_5$. |

European Patent Office

# EUROPEAN SEARCH REPORT

Application Number

**EP 90 40 2695**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | EP-A-0 274 025 (SRI INTERNATIONAL)<br>* Abstract; pages 6-11, structures 4-11,14-19; claims * | 1 | C 07 H 19/10<br>C 07 H 19/20<br>A 61 K 31/70 |
| X | DE-A-2 009 834 (SYNTEX CORP.)<br>* Page 1, lines 1-11; page 11, lines 6-9; pages 64-73; claims * | 1 | |
| Y | EP-A-0 354 246 (INSTITUT MOLEKULYARNOI BIOLOGII IMENI V.A. ENGELGARDTA AKADEMII NAUK S.S.S.R. et al.)<br>* Abstract * | 1 | |
| Y | WO-A-8 912 061 (MEDIVIR AB)<br>* Abstract * | 1 | |
| Y | EP-A-0 339 161 (MERRELL DOW PHARMACEUTICALS INC.)<br>* Claims * | 1 | |
| P,Y | EP-A-0 398 231 (BRISTOL-MYERS SQUIBB CO.)<br>* Abstract * | 1 | **TECHNICAL FIELDS SEARCHED (Int. Cl.5)** |
| Y | JOURNAL OF MEDICINAL CHEMISTRY, vol. 33, no. 9, 11th September 1990, pages 2481-2487, American Chemical Society; L. JIE et al.: "5'-O'-phosphonomethyl-2',3'-dideoxynucleosides: Synthesis and anti-HIV activity"<br>* Whole document * | 1 | C 07 H 19/00<br>A 61 K 31/00 |

The present search report has been drawn up for all claims

| Place of search | Date of completion of search | Examiner |
|---|---|---|
| The Hague | 28 May 91 | SCOTT J.R.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document